# EUROPEAN PATENT APPLICATION

(11) **EP 2 153 836 A1**
(43) Date of publication of application: **17.02.2010**
(21) Application number: 08161757.3
(22) Date of filing: 04.08.2008
(51) Int. Cl.: A61K 31/722, A61K 47/36, A61K 31/506, A61K 31/56, A61K 31/585, A61P 17/14

(54) **Film-forming liquid formulations for drug release to hair and scalp**

(71) Applicant: POLICHEM S.A., 1526 Luxembourg (LU)
(72) Inventor: Mailland, Federico, I-22100, Como (IT); Mura, Emanuela, I-22100, Como (IT)
(74) Representative: Pistolesi, Roberto

(57) **Abstract**

The present invention is directed to liquid compositions containing chitosan, a chitosan derivative or a physiologically acceptable salt thereof, forming a film after application onto the scalp and/or the hair, useful for delivery of actives onto the scalp surface and on the hair.

## Description

The present invention relates to liquid compositions containing chitosan, a chitosan derivative or a physiologically acceptable salt thereof, for the preparation of a medicament, or a medical device, or a sanitary product, or a cosmetic, forming a film after application onto the scalp, or onto the hair, useful for delivery of actives onto the scalp surface and on the hair.

Delivery of active ingredients to scalp or the hair, contained in drugs, sanitary products, detergents or cosmetics, often constitutes a problem in that the common formulations, like cream, ointment, gel, powder, or foam, do not allow a long lasting contact with the scalp or hair surface.

Chitosan and its derivatives are amino-polysaccharides, derived from the chitin extracted from the exoskeleton of the crustaceans, known in the art for their use in different preparations. KR20020084672 discloses chitosan as an ingredient of microspheres, useful as a carrier for separation of proteins or peptides; KR20020048534 reports chitosan as an ingredient of a pack composition for skin massage, including paraffin wax as an effective component; JP2005306746 is teaching the use of chitosan to obtain a wrinkle therapeutic agent as an ingredient of gel-like or spongy preparations of botulinus toxin. W02005055924 reports chitosan derivatives as ingredients of hydrogels useful for cavity-filling wound dressings. JP2004231604 teaches compositions of chitosans having a high deacetylation degree, as an ingredient of a carrier sheet with a porous spongy texture. W003042251 discloses compositions comprising chitosan in the form of a network of nano-sized fibres. W002057983 discloses a multi-layered, air gap sheet of chitosan with a regular lamellar structure which retains drugs for a prolonged period of time; JP11060605 teaches an amphiphilic chitosan derivative which can be used as dispersion stabilizer or emulsifier in a drug for application to skin. Finally, EP1303249, discloses a nail varnish composition containing at least one antimycotic agent and an hydroxyalkyl or a carboxyalkyl chitosan, whereas WO2004/112814 discloses a nail restructuring composition based on one herb extract from the genus Equisetum in combination with hydroxypropyl chitosan.

It has now surprisingly been found that liquid preparations of chitosan derivatives, besides being useful for the application to nails, may form an elastic film, after application to the scalp and after evaporation of the volatile solvents, suitable to maintain drugs and other actives in intimate contact to the scalp and/or hair surface. The film forming solutions containing chitosans, pharmaceutically active agents and volatile solvents, may easily be sprayed onto the scalp or hair surface, by allowing quick evaporation of the volatile solvents and easy formation of an elastic film, that avoids, as an example, bothersome sensation of oily scalp compared to creams, lotions and ointments, and avoids long time waiting for drying, when large scalp surface is to be treated. The film forming solutions of chitosan or chitosan derivatives may also be applied on the scalp by gently massage, or by spray. The film forming solutions of chitosans allow quick penetration of actives into the deep layers of the scalp, thus resulting useful for the safe treatment and/or prevention of scalp conditions and/or diseases, like hair loss, where penetration of the active ingredient until the hair follicles is needed. Moreover, the dermal film formed after evaporation of solvents of liquid chitosan compositions allows long lasting intimate contact with the scalp and/or hair surface, and continuous release of drugs or other actives for many hours after the application.

### DESCRIPTION OF THE INVENTION

The object of the present invention is a composition in form of a liquid formulation containing chitosan, a chitosan derivative or a physiologically acceptable salt thereof, useful for delivery of actives onto the scalp and/or hair. Among chitosan derivatives, hydroxyalkyl chitosans, such as hydroxypropyl chitosan, or other water soluble chitosans are preferred.

The film forming solutions of chitosan may be applied by a gently massage on the scalp or may easily be sprayed by allowing the formation of an elastic film. The film forming solutions of chitosans allow quick penetration of actives, a long lasting intimate contact with the scalp and continuous release of drugs for many hours after the application.

Liquid preparations object of this invention are in the form of solutions, emulsions, colloids or suspensions, with a content in chitosan derivative from 0.1 to 10 wt.% (percentages by weight are given with respect to the whole preparation), more preferably from 0.2 to 5 wt.%, most preferably from 0.25 to 2.0 wt.%; they contain at least one active pharmaceutical or cosmetic agent, or a plant extract, from 0.001 to 25 wt.%, more preferably from 0.2 to 10 wt.%, most preferably from 0.4 to 5.0 wt.%, suitable to form an elastic film after evaporation of solvent, said film being in intimate contact with the scalp surface and/or of the hair and allowing quick and long lasting penetration of said actives useful for treating scalp and/or hair conditions and/or diseases, like hair loss, baldness, alopecia, androgenetic alopecia, hair fragility and other hair conditions; they also contain at least one volatile solvent in an amount of from 25 to 90 wt.%, preferably from 30 to 85 wt.%, more preferably from 35 to 80 wt.%.

Compositions object of this invention are superior to the conventional formulations, in that they can be sprayed onto the surface, leaving an uniform and invisible film. Moreover, compositions according to the present invention do not dirty, do not dry like gels and lotions do, and do not give bothersome sensation when applied, like other rigid film preparations do.

Pharmaceutical compositions are prepared according to conventional technique, using compatible excipients and pharmaceutically acceptable carriers, and may contain, in combination, other active principles with complementary or, in any case, useful activity.

Examples of these compositions prepared according to the present invention include: solutions, emulsions, suspensions, colloids, for application to nude or hairy scalp.

The compositions according to the present invention may contain one or more active agents selected from 5-alpha reductase inhibitors, antiandrogenic hormones, potassium chanel agonists, aminoacids, plant extracts, antioxidants, and are suitable to prevent and to treat hair loss, baldness, alopecia; to nourish, volumize and reinforce the hair.

Examples of 5-alpha reductase inhibitors which may be included in the composition in accordance with the present invention include: finasteride, dutasteride, azelaic acid, betasitosterol, zinc, Vitamin B6.

Examples of antiandrogenic hormones which may be included in the composition in accordance with the present invention include: spironolactone, ciproterone acetate, flutamide, ketoconazole, oestrogens and their salts.

Examples of potassium chanel agonists which may be included in the composition in accordance with the present invention include minoxidil.

Examples of aminoacids which may be included in the composition in accordance with the present invention include: L-cysteine, N-acetylcisteine, L-cystine, L-methionine, dimethylsulphone, L-taurine.

Examples of plants which extracts may be included in the composition in accordance with the present invention include: *Serenoa repens, Aloe vera, Equisetum arvense, Panicum miliaceum, Pygeum africanum, Urtica dioica, Coix lachrymal-jobi, Eriobotrya Japonica.*

Examples of antioxidants which may be included in the composition in accordance with the present invention include: ascorbic acid; glutathione; melatonin; tocopherols and tocotrienols; polyphenolic antioxidants including resveratrol and flavonoids; viniferols; carotenoids including lycopene, carotenes.

The compositions according to the invention may be applied onto the scalp surface by gently massage of the concerned area, or onto the hair by spray. After evaporation, an elastic film is formed onto the treated surface, that allows the continuous delivery of the actives to the scalp and/or the hair for many hours or even for days.

Compositions object of this invention may also contain penetration modifying systems, including absorption enhancers, or modified delivery systems, which modify the penetration rate of the actives into the epidermis and create a reservoir at the dermal-epidermal junction, to provide an increased and longer lasting availability of the active at the hair follicles. Examples of the absorption enhancers which may be included in the composition in accordance with the present invention include Transcutol P® (Diethyleneglycole monoethyletere), liposomes. Examples of modified delivery systems which may be included in the composition in accordance with the present invention include microcapsules.

The pharmaceutical compositions and the uses of the present invention will now be more fully described by the following examples. It should, however, be noted that such examples are given by way of illustration and not of limitation.

### EXAMPLE 1

A film forming solution having the following composition wt./wt.% was prepared:

| | | |
|---|---|---|
| 1) | Finasteride | 0.25% |
| 2) | Ethyl Alcohol 96° | 55.00% |
| 3) | Propylene Glycol | 5.00% |
| 4) | Hydroxypropyl Chitosan | 1.00% |
| 5) | Purified water | 38.75% |

### Preparation

Ethyl Alcohol, Propylene Glycol and water were mixed at room temperature. Finasteride was then added and mixed until a clear solution was obtained. Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 2

A liquid formulation having the following w/w % composition was prepared:

| | | |
|---|---|---|
| 1) | Dutasteride | 0.25% |
| 2) | Ethyl Alcohol 96° | 55.00% |
| 3) | Propylene Glycol | 5.00% |
| 4) | Hydroxypropyl Chitosan | 2.00% |
| 5) | Purified water | 37.75% |

### Preparation

The formulation was prepared by using the same method described for Example 1.

Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 3

A liquid formulation having the following w/w % composition was prepared:

| | | |
|---|---|---|
| 1) | Minoxidil | 2.00% |
| 2) | Ethyl Alcohol 96° | 55.00% |
| 3) | Propylene Glycol | 5.00% |
| 4) | Hydroxypropyl Chitosan | 1.00% |
| 5) | Purified water | 37.00% |

### Preparation

The formulation was prepared by using the same method described for Example 1.

Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 4

A liquid formulation having the following w/w % composition was prepared:

| | | |
|---|---|---|
| 1) | Spironolactone | 1.00% |
| 2) | Ethyl Alcohol 96° | 55.00% |
| 3) | Propylene Glycol | 5.00% |
| 4) | Hydroxypropyl Chitosan | 2.00% |
| 5) | Purified water | 37.00% |

### Preparation

The formulation was prepared by using the same method described for Example 1.

Hydroxypropyl Chitosan was added as the final ingredient, and the mixture was stirred at room temperature for 24 hours or until dissolution.

The obtained formulation was a clear and colourless solution, homogenous in appearance even after prolonged storage. Moreover, the liquid was able to form a matte, non-sticky and elastic film, which could strongly adhere to the scalp surface.

### EXAMPLE 5

An *in vitro* permeation test was performed by applying the film forming solution according to the Example 1 to excised hairless rat skin, obtained from dorsal or abdominal skin of male hairless rats. Portions of the skin (ca 9 cm²), after removal of adhering fat and subcutaneous tissues, were placed as a barrier between the two compartment of Gummer permeation vertical cells (Gummer, C.L. et al. The skin penetration cell: design update. Int. J. Pharm. 1987, 40, 101-104). The receiving phase was introduced into the lower compartment and 1.0 or 0.5 mL of the composition according to the Example 1 were regularly distributed on the exposed skin surface. At predetermined time intervals (2, 4, 8, 12, 16, 20 and 24 hours) 5.0 mL of the receiving solution were collected for analysis and immediately replaced by an equal volume of fresh buffer. The experiment was replicated 3 times.

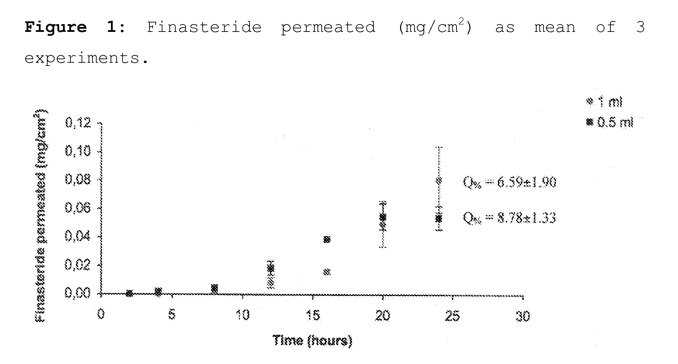

The finasteride permeated through hairless rat skin in the 3 experiments is reported in Figure 1. The total percent quantity (Q%) permeated through the rat hairless skin was 6.59 ± 1.90% for the 1.0 mL dose and 8.78 ± 1.33% for the 0.5 mL dose.

It is concluded that finasteride was able to permeate the rat skin, after the application of the film forming solution of hydroxypropyl chitosan according to the Example 1, in a quick and long lasting way.

### EXAMPLE 6

A preparation having the following composition wt./wt.% was prepared:

| | | |
|---|---|---|
| 1. | finasteride | 0.25% |
| 2. | purified water | 19.25% |
| 3. | propylene glycol | 10.00% |
| 4. | isopropanol | 70.00% |
| 5. | chitosan | 0.50% |

### Preparation

The formulation was prepared by dissolving chitosan and finasteride in propylene glycol, then adding the other ingredients, and stirring the mixture until dissolution. The resulting liquid was able to form an elastic film which could strongly adhere to the skin surface.

### EXAMPLE 7

A liquid formulation having the following w/w % composition was prepared:

| | | |
|---|---|---|
| *1.* | *Eriobotrya Japonica Leaf 1,3-butylene glycol extract*¹ | 10.00% |
| 2. | Ethyl Alcohol 96° | 36.50% |
| 3. | Diethyleneglycole monoethyletere² | 0.50% |
| 4. | Hydroxypropyl Chitosan | 1.00% |
| 5. | Purified water | 52.00% |

| | | |
|---|---|---|
| ¹ Loquat Leaf Extract CA; ²Transcutol^{®} P | | |

### Preparation

Ethyl Alcohol and water were mixed at room temperature.

The Loquat Leaf Extract CA was then added and mixed until a clear solution was obtained. Diethyleneglycole monoethyletere was added; Hydroxypropyl Chitosan was added as the final ingredient, and the mixture was stirred at room temperature for 4 hours or until dissolution.

The obtained formulation was a clear and colourless solution, homogenous in appearance. Moreover, the liquid was able to form a matte, non-sticky and elastic film.

### EXAMPLE 8

A liquid formulation having the following w/w % composition was prepared:

| | | |
|---|---|---|
| *1.* | *Coix lachrymal-jobi 1,3-butylene glycol solution*¹ | 10.00% |
| 2. | Ethyl Alcohol 96° | 36.50% |
| 3. | Diethyleneglycole monoethyletere¹ | 0.50% |
| 4. | Hydroxypropyl Chitosan | 1.00% |
| 5. | Purified water | 52.00% |
| ¹Hydrolyzed Coix Extract; ²Transcutol^{®} P | | |

### Preparation

Ethyl Alcohol and water were mixed at room temperature. The Hydrolyzed Coix Extract was then added and mixed until a clear solution was obtained. Diethyleneglycole monoethyletere was added; Hydroxypropyl Chitosan was added as the final ingredient, and the mixture was stirred at room temperature for 4 hours or until dissolution.

The obtained formulation was a clear and slightly yellow solution, homogenous in appearance. Moreover, the liquid was able to form a matte, non-sticky and elastic film.

## Claims

1. A composition containing:
(a) a chitosan, a chitosan derivative or a physiologically acceptable salt thereof,
(b) at least a pharmaceutical or a cosmetic active ingredient, and
(c)at least one volatile solvent,
for the treatment and/or prevention of scalp and/or hair conditions and/or diseases.

2. A composition according to claim 1, wherein component a) is present in an amount of from 0.1 to 10%, preferably from 0.2 to 5%, more preferably from 0.25 to 2.0%, with respect to the total weight of the composition.

3. A composition according to any of the preceding claims, wherein component a) is a water soluble chitosan derivative or a salt thereof.

4. A composition according to claim 3, wherein said water soluble chitosan derivative is an hydroxyalkyl-chitosan.

5. A composition according to claim 4, wherein said hydroxyalkyl-chitosan is hydroxypropyl-chitosan.

6. A composition according to any of the preceding claims, wherein component b) is present in an amount of from 0.001 to 25%, preferably from 0.2 to 10%, more preferably from 0.4 to 5.0%, with respect to the total weight of the composition.

7. A composition according to any of the preceding claims, wherein component b) is selected from 5-alpha reductase inhibitors, antiandrogenic hormones, potassium chanel agonists, aminoacids, plant extracts and antioxidants.

8. A composition according to any of the preceding claims, wherein component c) is present in an amount of from 25% to 90%, preferably from 30% to 85%, more preferably from 35% to 80%, with respect to the total weight of the composition.

9. A composition according to any of the preceding claims, wherein component c) is a lower alkanol, preferably selected from ethanol or isopropanol.

10. A composition according to any of the preceding claims, **characterized by** comprising water.

11. A composition according to any of the preceding claims, **characterized by** comprising customary excipients and/or adjuvants.

12. A composition according to any of the preceding claims, **characterized by** being liquid, preferably a solution, emulsion, suspension or colloid.

13. A composition according to any of the preceding claims, **characterized by** being applied by spray.

14. A composition according to any of the preceding claims, wherein said scalp and/or hair conditions and/or diseases are selected from hair loss, baldness, alopecia, androgenetic alopecia and/or hair fragility.

15. A composition according to one of the preceding claims, further containing at least a penetration modifying system, preferably selected from Diethyleneglycole monoethyletere, liposomes or microcapsules.
